Europäisches Patentamt

(19) European Patent Office

Office européen des brevets

(11) Veröffentlichungsnummer: **0 074 981**
**B1**

(12) **EUROPÄISCHE PATENTSCHRIFT**

(45) Veröffentlichungstag der Patentschrift:
04.09.85

(21) Anmeldenummer: **82900937.2**

(22) Anmeldetag: **01.04.82**

(86) Internationale Anmeldenummer:
**PCT/DE 82/00078**

(87) Internationale Veröffentlichungsnummer:
**WO 82/03323 (14.10.82 Gazette 82/25)**

(51) Int. Cl.⁴: **A 61 F 2/30**

(54) GELENKPROTHESE.

Verbunden mit 82730043.5/006199 (europäische
Anmeldenummer/Veröffentlichungsnummer) durch
Entscheidung vom 07.06.84.

(30) Priorität: **01.04.81 DE 3113531**
**23.10.81 DE 3142730**

(43) Veröffentlichungstag der Anmeldung:
**30.03.83 Patentblatt 83/13**

(45) Bekanntmachung des Hinweises auf die Patenterteilung:
**04.09.85 Patentblatt 85/36**

(84) Benannte Vertragsstaaten:
**AT BE CH DE FR GB IT LI NL SE**

(56) Entgegenhaltungen:
**CH - A - 389 231**
**DE - A - 2 305 441**

(73) Patentinhaber: **MECRON MEDIZINISCHE PRODUKTE
GMBH, Nunsdorfer Ring 25-27, D-1000 Berlin 48 (DE)**

(72) Erfinder: **KRANZ, Curt, Landshuter Strasse 5,
D-1000 Berlin 30 (DE)**

(74) Vertreter: **Christiansen, Henning, Dipl.-Ing., Unter den
Eichen 108a, D-1000 Berlin 45 (DE)**

### Beschreibung

Die Erfindung betrifft eine in der orthopädischen Chirurgie häufig angewendete Endoprothese (Gelenkprothese). Sie ist für alle Arten von Endoprothesen anwendbar, obgleich sich die nachfolgende Beschreibung eines Ausführungsbeispiels ausschliesslich auf eine Hüftgelenkprothese bezieht.

Die verschiedensten Indikationen können es angebracht erscheinen lassen, einem Patienten einen Gelenkersatz zu implantieren. Die herkömmlichen Endoprothesen haben aber nur eine Lebensdauer von etwa fünf bis acht Jahren. Die häufigste Versagensform dieser Prothesen ist die Lockerung aus dem Zementköcher bzw. bei zementlos eingesetzten Prothesen aus der Kortikalis des Femur. Die herkömmliche zementlos eingesetzte Endoprothese hat den Nachteil, dass der in den Knochen (z.B. Femur) hineinragende Schaft durch die notwendige Konfektionierung in einer bestimmten Anzahl von Grössen und durch das Fehlen einer weiteren Krümmung aus der einzigen Krümmungsebene heraus (keine Unterscheidung von rechts und links) ungenügend an die Form des Markhohlraums angepasst ist. Die daraus resultierenden punktuellen Auflagen führen zu örtlich sehr hohen Querbelastungen sowie Entlastungen der Kortikalis in Längsrichtung und damit zu Resorptionen im oberen Bereich des Prothesenansatzes. Die zementierte Endoprothese ist zwar durch den Zement formschlüssig in dem Hohlraum verankert, jedoch stellt der Verbund zwischen Prothesenschaft und Zementköcher ein um etwa eine Zehnerpotenz biegesteiferes Bauteil dar als die Kortikalisröhre des Knochens. Die Folge ist eine Umorientierung des Kraftflusses in der Kortikalis mit einer grossflächigen Entlastung sowie örtlichen Überbelastungen quer zur Faserorientierung.

Dementsprechend liegt der Erfindung die Aufgabe zugrunde, eine Gelenkprothese zu schaffen, die in Form und Steifigkeitsverteilung ohne wesentliche Kompromisse an die Kortikalis des Knochens individuell angepasst werden kann und in der Lage ist, die für den allgemeinen Bewegungsablauf erforderlichen Kräfte sowohl statisch als auch dynamisch zu ertragen und auf physiologische Weise in die Kortikalis einzuleiten, so dass Lockerungen in geringerem Masse zu erwarten sind als bei herkömmlichen Prothesen.

Diese Aufgabe wird gelöst durch die Gelenkprothese mit den Merkmalen des Patentanspruches 1. Vorteilhafte Weiterbildungen sind in den Unteransprüchen angegeben. Der dabei verwendete Begriff «Prepreg» ist die übliche Bezeichnung für bereits mit einem Matrix-Kunststoff benetzte Faser-Gelege (Glas, Kohle, Aramid od. ähnliches), die noch nicht ausgehärtet sind, d.h. deren Polymerisationsprozess erst durch äussere Einflüsse wie Wärme, ultraviolettes Licht, Licht, Ultraschall oder ähnliches angeregt werden muss. Der dabei verwendete Matrix-Kunststoff ist ein Kunststoff, der in flüssiger Form mit Fasern oder Geweben in Verbindung gebracht wird und nach der Aushärtung einen Verbund zwischen den einzelnen Fibern bzw. Fasern herstellt. Diese sind in dem Gelege aufeinandergeschichtet oder in Gestalt gerichteter Gewebe mit dem Ziel enthalten, genau definierte, gerichtete Steifigkeiten sowie Festigkeiten des in Verbindung mit geeigneten Matrix-Kunststoffen sich ergebenden Verbundes zu erzeugen.

Bevorzugt besteht das Prepreg-Teil aus besonders zugeschnittenen endseitig befestigten Streifen, welche die tragende und krafteinleitende Funktion der Prothese übernehmen und erst während der Operationsphase durch die Verwendung von ultraviolettem Licht oder Ultraschall ausgehärtet werden und damit exakt an die Kortikalis angepasst werden. Die Kraftüberleitung auf einen Gelenkkopf, beispielsweise eine Aluminiumoxid-Keramikkugel geschieht durch ein besonders geformtes Anpassteil (Adapter), das bevorzugt aus einer körperveträglichen Titanlegierung besteht. Durch die Anwendung eines bereits in der Zahnmedizin gebräuchlichen Kunststoffs, der durch ultraviolettes Licht aushärtet, zum Einbetten der Fasern der Prepreg-Streifen entstehen keine zusätzlichen Komplikationen im Zusammenhang mit der Körperverträglichkeit des gesamten Implantats. Vielmehr ist gleiche oder bessere Verträglichkeit mit dem menschlichen Körper zu erwarten als bei herkömmlichen Prothesen. Alle für die Implantation notwendigen Hilfsstoffe und Vorrichtungen können aus gebräuchlichen sterilisierbaren Stoffen gefertigt werden. Als Faserwerkstoff wird Glas, Aramid oder bei Aushärtung durch Ultraschall auch Carbon verwendet.

Die grössere Lebensdauer durch verminderte Gefahr der Lockerung und der damit verbundenen Bruchgefahr bei herkömmlichen Prothesen verdankt die erfindungsgemässe Gelenkprothese der besseren individuellen Anpassbarkeit der tragenden und krafteinleitenden Struktur des Implantats (Prepreg-Teil) an die physiologischen Gegebenheiten (Form, Rauhigkeit) des Markhohlraums während des Einsetzens der Gelenkprothese im Verlauf einer Operation sowie der an die physiologischen Bedingungen (Biegesteifigkeit) besser angepassten Krafteinleitung in die Kortikalis des Knochens bei weitgehender Vermeidung von unphysiologischen Kräften senkrecht zur Faserorientierung der Kortikalis. Dabei ist die neue Gelenkprothese ohne wesentlich grösseren Aufwand zu implantieren als die herkömmlichen Prothesen, und sie ist im Falle einer eventuellen notwendigen Reoperation ohne grossen Aufwand zu entfernen. Sie ist in allen Fällen einsetzbar, in denen derzeit die konventionelle Form der Prothese benutzt wird, und im besonderen, wenn eine physiologische Abnormität die Einpassung einer konventionellen Prothese erschwert oder unmöglich macht, weil diese nur in bestimmten Abmessungen konfektioniert auf dem Markt angeboten wird. Durch hohe Anpassungsfähigkeit ist das Anwendungsspektrum bedeutend weiter als das der herkömmlichen Prothesenform.

Anhand der Zeichnungen wird ein bevorzugtes Ausführungsbeispiel der Erfindung beschrieben, bei welchem das Prepreg-Teil und eine Anpressmittel durch voneinander getrennte Teile gebildet sind, während die Einführhilfe und das Einführungsteil für das Aushärtemittel durch ein gemeinsames, beide Funktionen erfüllendes Teil gebildet sind, das hohl ist und durchlässig, sowohl für ein Strömungsmittel zum Aufblähen der Anpressmittel als auch für Strahlung von einem zum Aushärten des Prepreg-Teiles vorgesehenen Strahler, der in dem hohlen Teil, das zugleich Einführhilfe und Einführungsteil ist, geführt ist. Es ist ersichtlich, dass die Art der Zuordnung verschiedener Funktionen zu unterschiedlichen bzw. gemeinsamen Teilen, wie es im folgenden beschrieben und dargestellt ist, nur eine der möglichen Ausführungsformen wiedergibt. Es zeigen:

Fig. 1 im Längsschnitt dargestellt eine Gelenkprothese als Ausführungsbeispiel der Erfindung,

Fig. 2 die Hauptbestandteile derselben Gelenkprothese, die in eine Kortikalis eingesetzt, dort aber noch nicht befestigt ist,

Fig. 3 die Anordnung nach Fig. 2, jedoch während des Aushärtens, und

Fig. 4 die fertig eingesetzte und vervollständigte Gelenkprothese.

In den untereinander mit identischen Bezugszeichen versehenen Figuren ist übereinstimmend die folgende Anordnung vorhanden:

Ein zweiteiliges Anpassteil 1, welches eine Klemmverbindung mit einem Prepreg-Teil 2 bildet, ist mit einem Halteteil 1a versehen, auf dessen Gewindehals 1b ein Überwurfteil 1c aufgeschraubt ist. Das Prepreg-Teil 2 ist mit seinem oberen Bereich zwischen den Teilen 1a und 1c eingespannt. Eine durch den Gewindehals des Anpassteils 1 hindurchgeführte, in das Prepreg-Teil hineinragende, Einführhilfe 3 stellt zugleich das sogenannte Einführungsteil dar, auf das verschiedentlich getrennt Bezug genommen wird. Anpressmittel 4 sind im Inneren des Prepreg-Teils in Gestalt eines Folienbeutels und eines am unteren Ende des Einführungsteiles 3 zusammen mit diesem verschlossenen, aufblähbaren Schlauches vorgesehen. Ein in das Einführungsteil hineinführbarer UV-Licht- oder Ultraschall-Strahler 5 ist mit Transportmitteln 6 versehen. Weiterhin sind ein Rohr 7, Dichtungen 8 und 9 und eine Montagehilfe 10 mit einem Stutzen 10a vorgesehen. Die Montagehilfe 10 besteht aus einem aufschraubbaren Adapter, welcher die notwendigen Anschlüsse zum Durchlass von Strömungs- und Aushärtemitteln aufweist. Durch die Montagehilfe ist wegen der vergrösserten zugänglichen Oberfläche die Handhabung der Prothese bei der Implantation erleichtert.

Das Prepreg-Teil 2 besteht aus Längsstreifen und/oder gegenläufigen spiraligen Streifen, die auf geeignete Weise verwoben sind, die zwischen dem Halteteil 1a und dem aufgeschraubten Überwurfteil 1c eingeklemmt sind und auf diese Weise den Eindruck einer gefiederten Glocke erwecken, die jedoch — falls die Aushärtung nicht auch hier erst in der Operationsphase erfolgt — in einem oberen Bereich, wo sie am Anpassteil 1 befestigt ist, ausgehärtet ist, während sie in einem unteren Bereich 2b noch verformbar und unausgehärtet vorliegt.

In dem Gewindehals 1b steckt ein Rohr 7, in welches wiederum die Einführhilfe 3 so eingesteckt ist, dass das weitgehend für Strömungsmittel undurchlässige, insbesondere luftdichte und aufblähbare Anpressmittel 4 an dessen oberen, dem Anpassteil 1 zugewandten Ende eingeklemmt ist. Zur Aufblähung dieses Anpressmittels 4, z.B. Folien- oder Gummisackes, kann durch den Stutzen 10a Luft durch die

Montagehilfe 10, das Rohr 7, die Einführhilfe 3 und darin vorgesehene Löcher 3a in die Anpressmittel 4 geleitet werden. Um das Entweichen dieser Luft an unerwünschten Stellen zu verhindern, sind die Dichtungen 8 und 9 vorgesehen. Zum Gewährleisten des Entweichens der Luft aus dem Hohlraum 11 ist eine nicht näher dargestellte Entlüftung in dem Anpassteil 1 vorgesehen, durch welche auch Luft (zusätzlich oder anstelle der Druckluftanwendung über den Stutzen 10a) abgesaugt werden kann. Die in die Anpressmittel 4 gedrückte und/oder aus dem Hohlraum 11 gesaugte Luft ist das im vorliegenden Ausführungsbeispiel anzuwendende Strömungsmittel, durch welches die Anpressmittel 4 aufblähbar sind, um die Streifen des Prepreg-Teiles 2 im Bereich 2b gegen die Knocheninnenwand zu pressen. Um diesen angepassten Zustand zu konservieren, ist ein Aushärtemittel vorgesehen, nämlich der Strahler 5.

Zum Einführen ist das Einführungsteil 3 in Gestalt eines Rohres aus Polyäthylen oder ähnlichem vorgesehen, das zugleich als Einführhilfe für die noch flexiblen Teile 2 (im Bereich 2b) und 4 dient.

Als Transportmittel 6 für den Strahler 5 dient ein längliches, federnd nachgiebiges Element, mittels dessen dem Strahler Energie zugeführt wird, entweder in Gestalt elektrischer Energie oder von ultraviolettem Licht, wenn das Transportmittel 6 zugleich als Lichtleiter ausgebildet ist. Der Transport des Strahlers 5 erfolgt mit definierter Ausziehgeschwindigkeit durch nicht dargestellte Antriebsmittel, die an der Montagehilfe 10 befestigt sein können. Die Prepreg-Streifen im Bereich 2b können so ausgebildet sein, dass dazwischen Leerräume verbleiben, wenn sie an eine Knocheninnenwand angedrückt sind. In diese Zwischenräume kann dann später Knochengewebe einwachsen. Als Kunststoffmaterial der Prepregs kann z.B. ein Material verwendet werden, welches unter der Bezeichnung «UVIOBOND»® in der zahnärztlichen Praxis Verwendung findet.

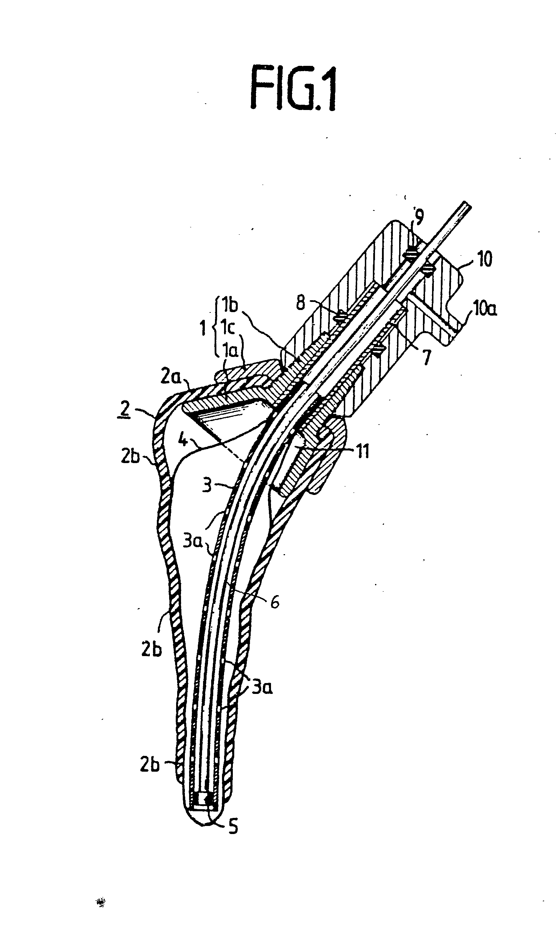

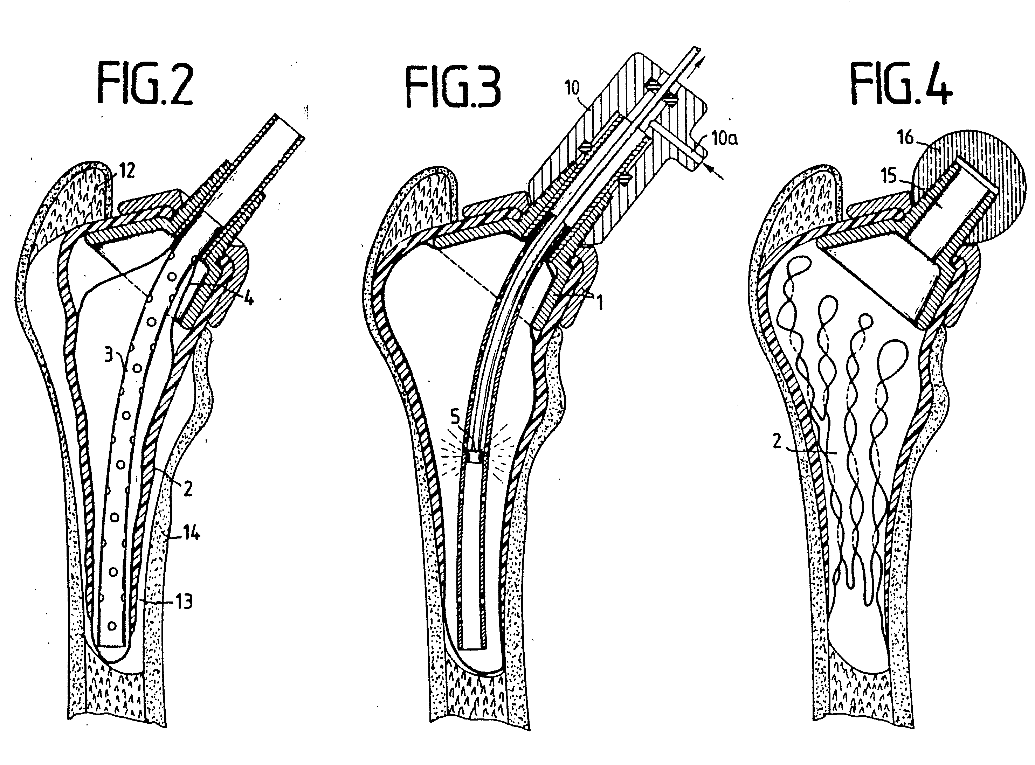

In Fig. 2 ist gezeigt, wie die Gelenkprothese nach Fig. 1 nach weitgehendem Ausräumen des Spongiosa 12 aus einem Markhohlraum 13 einer Kortikalis 14 eingesetzt ist.

Fig. 3 zeigt eine spätere Phase mit angepressten Prepreg-Streifen nach dem Eindrücken von Luft in den Stutzen 10a unter leichtem Überdruck von ungefähr 0,8 bar. Der UV-Licht- oder Ultraschall-Strahler 5 hat den Markhohlraum 13 schon zur Hälfte durchlaufen und durch seine die Teile 3 und 4 durchdringende Strahlung die Aushärtung eines Teils der Prepreg-Streifen 2 bewirkt. Dabei entsteht keine Wärme im Gegensatz zur Verwendung von Knochenzement. Die Aushärtung dauert ungefähr 6 bis 8 Minuten bei Verwendung von ultraviolettem Licht zur Aushärtung.

Nach Beendigung der in Fig. 3 gezeigten Phase werden die nicht mehr benötigten Hilfsmittel, also die Teile 3 bis 10 entfernt, wofür bezüglich der Teile 3 bis 7 ein Kanal (15 in Fig. 4) im Anpassteil 1 vorgesehen ist. Er ist von dem Gewindehals 1b (Fig. 1) umgeben, auf welchen die Montagehilfe 10 aufschraubbar ist für die Bewerkstelligung der Vorgänge des Anpressens des Prepreg-Teiles 2 und der Aushärtung. Nach dem Aushärten wird die Montagehilfe 10 abgeschraubt und nach dem Entfernen der restlichen Hilfsmittel statt dessen ein Gelenkkopf 16, z.B. eine Aluminiumoxid-Keramikkugel aufgeschraubt, wie es in Fig. 4 zur Darstellung des Endzustandes gezeigt ist. Der Gewindehals 1b weist zu diesem Zweck ein selbsthemmendes, konisches Gewinde auf.

Der weitere Verlauf der Operation, einschliesslich des Einsetzens der Hüftpfanne, geschieht in der herkömmlichen Art und Weise.

Während bei den bekannten Hüftgelenkprothesen die Krafteinleitung in den Knochen unter Ausbildung von Querkräften geschieht, denen der Knochen in der Regel nicht gewachsen ist, erfolgt die Krafteinleitung bei der Gelenkprothese nach der Erfindung in der physiologisch richtigen Richtung, wobei die Prothese einerseits ein grosses Trägheitsmoment aufweist und andererseits die Einleitungskräfte pro Flächeneinheit wegen der grossen beteiligten Flächen gering sind. Querbelastungen des Knochens und eine mehr oder weniger punktförmige Kraftübertragung wie bei herkömmlichen Prothesen werden vermieden, zumal die Struktur selbst elastisch ist und damit den elastischen Verformungen des Knochens folgen kann.

Eine Reparatur der erfindungsgemässen Prothese ist im Gegensatz zu den bekannten Prothesen durch Ersatz einzelner Teile möglich. Darüber hinaus kann später eine neue Prothese wegen des zur Verfügung stehenden Hohlraumes ohne weiteres eingebracht werden, wobei diese herkömmlicher Art sein oder auch der erfindungsgemässen Ausführungsform entsprechen kann.

**Patentansprüche**

1. Gelenkprothese, gekennzeichnet durch folgende Bestandteile:
   a) wenigstens ein Anpassteil (1),
   b) wenigstens ein mit dem Anpassteil (1) verbundenes und in denjenigen Bereichen (2b) aushärtbares Prepreg-Teil (2), die dem Knochenhohlraum (13) anzupassen sind, in den die Gelenkprothese einzusetzen ist,
   c) für ein Strömungsmittel, weitgehend undurchlässige und durch dieses aufblähbare Anpressmittel (4) zum Anpressen der noch unausgehärteten Bereiche (2b) des Prepreg-Teiles (2) mittels des zuführ- und/oder absaugbaren Strömungsmittels.

2. Gelenkprothese nach Anspruch 1, gekennzeichnet durch wenigstens eine Einführhilfe (3), welche in die weitgehend für Strömungsmittel undurchlässigen Anpressmittel (4) einführbar oder in diese eingeführt und steifer ausgestaltet ist als die Anpressmittel (4).

3. Gelenkprothese nach einem der vorangehenden Ansprüche, dadurch gekennzeichnet, dass das Prepreg-Teil (2) mindestens in demjenigen Bereich (2a), in dem es vom Anpassteil (1) gehalten ist, schlauch- oder ring-, insbesondere glockenförmig ist und/oder bereits vorausgehärtet ist.

4. Gelenkprothese nach einem der vorangehenden Ansprüche, dadurch gekennzeichnet, dass das Prepreg-Teil (2) mindestens am noch unausgehärteten Ende streifenförmige und/oder in Form von wechselnd gegenläufigen Spiralen, die miteinander verwoben sind, ausgebildete Bereiche (2b) aufweist.

5. Gelenkprothese nach einem der vorangehenden Ansprüche, dadurch gekennzeichnet, dass ein Einführungsteil für Aushärtemittel (5) für die unausgehärteten Bereiche (2b) des Prepreg-Teiles (2) vorgesehen ist.

6. Gelenkprothese nach Anspruch 5, dadurch gekennzeichnet, dass das Einführungsteil zugleich als Einführhilfe (3) und/oder Einleitungsteil für das zuzuführende Strömungsmittel ausgebildet ist.

7. Gelenkprothese nach einem der vorangehenden Ansprüche, dadurch gekennzeichnet, dass das Aushärtemittel (5) als UV-Licht- und/oder Ultraschall-Strahler ausgebildet ist und zwischen Strahler und Prepreg-Teil (2) befindliche Teile (3, 4) für die Strahlung durchlässig sind.

8. Gelenkprothese nach einem der Ansprüche 6 oder 7, dadurch gekennzeichnet, dass das Einführungsteil als Führungselement für den Strahler (5) ausgebildet ist.

9. Gelenkprothese nach einem der vorangehenden Ansprüche, dadurch gekennzeichnet, dass das Einführungsteil (3) derart hohl geformt ist, dass das Aushärtemittel (5) hindurchführbar ist.

10. Gelenkprothese nach einem der vorangehenden Ansprüche, dadurch gekennzeichnet, dass die für Strömungsmittel weitgehend undurchlässigen Anpressmittel (4) im aufgeblähten, aber nicht merklich gedehnten Zustand erweitert ausgebildet sind im Vergleich zu dem bestimmungsgemäss zur Aufnahme von Gelenkprothesenteilen vorgesehenen Knochenhohlraum (13) und/oder weniger weit ausgebildet sind als der bestimmungsgemäss zur Aufnahme von Gelenkprothesenteilen vorgesehene Knochenhohlraum (13) und/oder beutelförmig ausgebildet sind.

11. Gelenkprothese nach einem der vorangehenden Ansprüche, dadurch gekennzeichnet, dass die weitgehend für Strömungsmittel undurchlässigen Anpressmittel (4) aus Folie bestehen.

12. Gelenkprothese nach einem der vorangehenden Ansprüche, dadurch gekennzeichnet, dass das Prepreg-Teil (2) zugleich als weitgehend für Strömungsmittel undurchlässiges Anpressmittel (4) ausgebildet ist.

13. Gelenkprothese nach einem der vorangehenden Ansprüche, dadurch gekennzeichnet, dass das Anpassteil (1) einen Kanal (15) für die Einführhilfe (3) ringförmig umgibt und/oder ein Tragelement für Endoprothesenteile (Gelenkkopf 16) bildet.

14. Gelenkprothese nach Anspruch 13, dadurch gekennzeichnet, dass durch den Kanal (15) hindurch mindestens eines der folgenden Bauteile entfernbar ist: die Einführhilfe (3), das Einführungsteil, die Aushärtemittel (5), die Transportmittel (6) für die Aushärtemittel (5), die Anpressmittel (4).

15. Gelenkprothese nach einem der Ansprüche 13 oder 14, dadurch gekennzeichnet, dass der Kanal (15) innerhalb eines Stutzens (Gewindehals 1b) des Anpassteiles (1) angeordnet ist, der lösbar mit einer Montagehilfe (10) verbindbar ist.

**Claims**

1. A joint prosthesis characterised by the following components:

a) at least one adapter portion (1),

b) at least one prepreg portion (2) which is connected to the adapter portion (1) and which can be cured in those regions (2b) which are to be fitted into the bone cavity (13) in which the joint prosthesis is to be insered,

c) pressure means (4) which are largely impermeable to a flowing medium and can be inflated by this to press the still uncured regions (2b) of the prepreg portion (2) by means of the flowing medium which can be supplied and/or drawn off.

2. A joint prosthesis as claimed in claim 1, characterised by at least one insertion aid (3) which can be inserted in the pressure means (4) which are largely impermeable to flowing media or which is inserted in these pressure means and is stiffer in construction than the pressure means (4).

3. A joint prosthesis as claimed in any of the preceding claims, characterised in that the prepreg portion (2) is in the form of a flexible tube or ring, particularly in the form of a bell and/or is already precured, at least in that region (2a) in which it is held by the adapter portion (1).

4. A joint prosthesis as claimed in any of the preceding claims, characterised in that the prepreg portion (2) comprises regions (2b) which are constructed in the form of strips and/or in the form of alternating oppositely directed spirals, which are interwoven with one another, at least at the end which is not yet cured.

5. A joint prosthesis as claimed in any of the preceding claims, characterised in that an introduction member is provided for curing means (5) for the uncured regions (2b) of the prepreg portion (2).

6. A joint prosthesis as claimed in claim 5, characterised in that the introduction member is constructed, at the same time, as an insertion aid (3) and/or an introduction member for the flowing medium to be supplied.

7. A joint prosthesis as claimed in any of the preceding claims, characterised in that the curing means (5) is constructed in the form of an ultraviolet light radiator and/or ultrasonic radiator and parts (3, 4) situated between radiator and prepreg portion (2) are permeable to the radiation.

8. A joint prosthesis as claimed in claim 6 or 7, characterised in that the introduction member is constructed in the form of a guide element for the radiator (5).

9. A joint prosthesis as claimed in any of the preceding claims, characterised in that the introduction member (3) is hollow in shape such that the curing means (5) can be passed through it.

10. A joint prosthesis as claimed in any of the preceding claims, characterised in that the pressure means (4) which are largely impermeable to flowing media are constructed, in the inflated but not noticeably stretched state, widened in comparison with bone cavity (13) provided, according to requirements, for the reception of joint prosthesis parts and/or are less wide in construction than the bone cavity (13) provided, according to requirements, for the reception of joint prosthesis parts and/or are of bag-shaped construction.

11. A joint prosthesis as claimed in any of the preceding claims, characterised in that the pressure means (4) which are largely impermeable to flowing media consist of foil.

12. A joint prosthesis as claimed in any of the preceding claims, characterised in that the prepreg portion (2) is constructed at the same time as pressure means (4) which are largely impermeable to flowing media.

13. A joint prosthesis as claimed in any of the preceding claims, characterised in that the adapter portion (1) surrounds, in annular form, a passage (15) for the insertion aid (3) and/or forms a supporting element for endoprosthesis parts (articular head 16).

14. A joint prosthesis as claimed in claim 13, characterised in that at least one of the following components can be removed through the passage (15): the insertion aid (3), the introduction member, the curing means (5), the conveying means (6) for the curing means (5), the pressure means (4).

15. A joint prosthesis as claimed in claim 13 or 14, characterised in that the passage (15) is disposed inside a socket (threaded neck 1b) of the adapter portion (1), which can be detachably connected to an installation aid (10).

**Revendications**

1. Prothèse d'articulation caractérisée par les composants suivants:

a) au moins une pièce d'adaptation (1),

b) au moins une pièce prepreg (2) liée à la pièce d'adaptation (1) et pouvant faire prise dans les zones (2b) qui doivent être adaptées au volume creux de l'os (13) dans lequel il faut insérer la prothèse d'articulation,

c) un moyen de pressage (4) largement imperméable au moyen d'écoulement et pouvant être gonflée par lui, pour presser les zones (2b) n'ayant pas encore fait prise, de la pièce prepreg (2) au moyen du moyen d'écoulement que l'on peut amener et/ou que l'on peut aspirer.

2. Prothèse d'articulation selon la revendication 1, caractérisée par au moins un auxiliaire d'introduction (3), que l'on peut introduire dans le moyen de pressage (4), largement imperméable au moyen d'écoulement, ou bien qu'il est introduit dans ce moyen de pressage et qui est de structure que le moyen de pressage (4).

3. Prothèse d'articulation selon l'une des revendications précédentes, caractérisée en ce que la pièce prepreg (2), au moins dans la zone (2a) dans laquelle elle est maintenue par la pièce d'adaptation (1), a la forme d'une chambre à air ou une forme annulaire, en particulier la forme d'une cloche, et/ou a déjà fait préalablement prise.

4. Prothèse d'articulation selon l'une des revendications précédentes, caractérisée en ce que la pièce prepreg (2) présente, à son extrémité n'ayant pas encore fait prise, des zones (2b) en forme de bandes et/ou en forme de spirales à pas alternativement opposé et entrelacées l'une à l'autre.

5. Prothèse d'articulation selon l'une des revendications précédentes, caractérisée en ce qu'il est prévu une pièce d'introduction pour moyen de prise (5) pour les zones (2b), n'ayant pas encore fait prise, de la pièce prepreg (2).

6. Prothèse d'articulation 5, caractérisée en ce que la pièce d'introduction est en même temps conçue comme auxiliaire d'introduction (3) et/ou comme pièce d'introduction pour le moyen d'écoulement qu'il faut y amener.

7. Prothèse d'articulation selon l'une des revendications précédentes, caractérisée en ce que le moyen de prise (5) est conçu comme émetteur de lumière ultraviolette ou d'ultra-sons et en ce que des pièces (3, 4) qui se trouvent entre l'émetteur et la pièce prepreg (2) sont perméables au rayonnement.

8. Prothèse d'articulation selon l'une des revendications 6 ou 7, caractérisée en ce que la pièce d'introduction est conçue comme élément de guidage pour l'émetteur (5).

9. Prothèse d'articulation selon l'une des revendications précédentes, caractérisée en ce que la pièce d'introduction (3) a une forme creuse de telle sorte que l'on peut y introduire le moyen de prise (5).

10. Prothèse d'articulation selon l'une des revendications précédentes, caracterisée en ce que les moyens de pressage (4), largement imperméables au moyen d'écoulement, ont une forme élargie à l'ètat gonflé mais non notablement allongé, en comparaison du volume creux de l'os (13) règlementairement prévu pour recevoir les pièces de la prothèse d'articulation et/ou sont prévus moins large que le volume creux de l'os (13) règlementairement prévu pour recevoir les pièces de la prothèse d'articulation et/ou ont la forme d'un sac.

11. Prothèse d'articulation selon l'une des revendications précédentes, caractérisée en ce que les moyens de pressage (4), largement imperméables au moyen d'écoulement, sont constitués d'une feuille.

12. Prothèse d'articulation selon l'une des revendications précédentes, caractérisée en ce que la pièce prepreg (2) est également conçue comme moyen de pressage (4) largement imperméable au moyen d'écoulement.

13. Prothèse d'articulation selon l'une des revendications précédentes, caractérisée en ce que la pièce d'adaptation (1) entoure annulairement un canal (15) pour l'auxiliaire d'introduction (3) et/ou forme un élément support pour les pièces de l'endoprothèse (rotule 16).

14. Prothèse d'articulation selon la revendication 13, caractérisée en ce que l'on peut sortir par le canal (15) au moins l'un des composants suivants: l'auxiliaire d'introduction (3), la pièce d'introduction, le moyen de prise (5), le moyen de transport (6) pour le moyen de prise (5), le moyen de pressage (4).

15. Prothèse d'articulation selon l'une des revendications 13 ou 14, caractérisée en ce que le canal (15) est disposé à l'intérieur d'une tubulure (col filété 1b) de la pièce d'adaptation (1), tubulure que l'on peut relier, de façon amovible à un auxiliaire de montage (10).

# FIG.1